# EUROPEAN PATENT SPECIFICATION

(11) **EP 3 975 924 B1**
(45) Date of publication and mention of the grant of the patent: **07.10.2026**
(21) Application number: 19931017.8
(22) Date of filing: 30.05.2019
(51) Int. Cl.: A61F 2/02, A61F 2/04

(54) **PROSTHESIS FOR THE LUNG**
PROTHESE FÜR DIE LUNGE
PROTHÈSE POUR LE POUMON

(43) Date of publication of application: 06.04.2022
(73) Proprietor: National Institute Of Biological Sciences, Beijing, Beijing 102206 (CN)
(72) Inventor: TANG, Nan, Beijing 102206 (CN); WU, Huijuan, Beijing 102206 (CN); LI, Jiao, Beijing 102206 (CN)
(74) Representative: Latscha Schöllhorn Partner AG
(86) International application number: PCT/CN2019/089356
(87) International publication number: WO 2020/237586

(56) References cited:
- CN-A- 101 172 063
- CN-A- 102 459 564
- CN-A- 105 232 180
- CN-A- 106 413 601
- US-A1- 2009 255 537
- US-A1- 2015 265 392
- US-A1- 2015 272 591
- US-B1- 10 258 457
- US-B1- 10 258 457
- US-B2- 6 653 525

## Description

### Introduction

Fibrosis-the thickening and scarring of connective tissue that can result from injury-is characterized by the accumulation of extracellular matrix (ECM) components and the excessive proliferation of fibroblast cells. This disorder, which is commonly observed in organs including lungs, livers, and kidneys, among many others, causes disrupted tissue architecture and leads to major impairments in organ function^{1,2} . Indeed, fibrosis can develop in nearly every organ and is a major cause of end-stage organ failure and death in a large variety of chronic diseases³ . A common feature of pulmonary fibrosis is the excessive proliferation of fibroblasts around the air sacs of lungs (alveoli)⁴. Extensive biomedical studies have established that an increased number of fibroblasts, in combination with their excessive deposition in the ECM ultimately cause alveolar structure destruction, decreased lung compliance, and disrupted gas exchange function⁵⁻⁷ .

The most common type of pulmonary fibrosis is idiopathic pulmonary fibrosis (IPF). This disorder eventually affects entire lung lobes, but it begins with microscopic fibrotic lesions that occur at the peripheral regions and slowly progress inward, and this fibrosis can ultimately lead to respiratory failure^{8,9} . IPF is a fatal disease with the median survival time of only 2-4 years from diagnosis¹⁰ . Scientifically, the mechanisms and nature of the pathological progression of IPF are not fully understood, although multiple studies have implicated contributions from a specific subset of alveolar epithelial cells-alveolar type II (AT2) cells¹¹⁻¹³ .

The alveolar epithelia of lungs are composed of a combination of both alveolar type I (AT1) and type II (AT2) cells. AT2 cells are the alveolar stem cells, and can differentiate into AT1 cells during alveolar homeostasis and post-injury repair^{14,15} . AT1 cells-which ultimately constitute fully 95% of the alveolar surface in adult lungs-are large squamous cells that function as the epithelial component of the thin air-blood barrier¹⁶ . In IPF tissues, abnormal hyperplastic AT2 cells are typically located adjacent to fibroblastic foci¹⁷ , and the gene mutants that affect the functions of AT2 cells are frequently observed in IPF tissues in the clinic^{11-13,18,19} . Previously, balloon prosthesis was used to occupy the pleural cavity in order to correct tracheal shift and overdistension of the remaining lung following pneumonectomy (PNX), and such balloon is preferably constructed approximately 10% larger than the pleural cavity it is intended to occupy at the end of a normal exhalation. Prostheses which are designed to occupy space in the pleural cavity are known from US 2015/272591, US 2015/265392, US 6653525 and US 10258457.

Although mechanical tension is an important regulator of lung formation, function, and metabolism, studies at the cellular and molecular levels have been limited by lacking suitable tools to study the functions of mechanical tension on cells *in vitro* or *in vivo.*

### Summary of the Invention

The invention relates to a prosthesis for treating pulmonary fibrosis, in particular, idiopathic pulmonary fibrosis (IPF) and is defined in the appended claims. The present invention is based on an essential regulatory role of mechanical tension in driving the development of lung fibrosis. The implanted prosthesis may reduce the level of mechanical tension on the alveolar epithelium, and rescue the enlarged alveoli phenotype. In particular, progressive lung fibrosis can be prevented by reducing the level of mechanical tension on the alveolar epithelium.

In the first place, the invention provides prosthesis for treating pulmonary fibrosis, in particular, idiopathic pulmonary fibrosis (IPF).

The prosthesis in the present invention is designed to be placed under the pulmonary lobe, as long as at the end of normal inspiratory, the level of mechanical tension on the alveolar epithelium is reduced.

The prosthesis is designed to be anchored under the lobes and above the diaphragm, and designed to match with the bottom of the lobes, as long as the mechanical tension on the alveolar epithelium is reduced at the end of normal inspiratory.

Therefore, the prosthesis is surrounded by pleural fluid. Preferably, the prosthesis will occupy at least 1/8 of the space surrounded by the bottom of the lobes, diaphragm and the wall of pleural cavity at the end of normal inspiratory. Preferably, the prosthesis will occupy at least 1/4 of the space surrounded by the basal of the lobes, diaphragm and the wall of pleural cavity at the end of normal inspiratory. Preferably, the prosthesis will occupy at least half of the space surrounded by the basal of the lobes, diaphragm and the wall of pleural cavity at the end of normal inspiratory. Preferably, the prosthesis will occupy at least 3/4 of the space surrounded by the basal of the lobes, diaphragm and the wall of pleural cavity at the end of normal inspiratory. Preferably, the prosthesis will occupy the whole space surrounded by the basal of the lobes, diaphragm and the wall of pleural cavity at the end of normal inspiratory.

Therefore, the prosthesis is surrounded by pleural fluid. Preferably, the area of the prosthesis projected to the bottom of the lower lung lobe will account for at least 1/8 of the area of the bottom of the lower lung lobe. Preferably, the area of the prosthesis projected to the bottom of the lower lung lobe will account for at least 1/4 of the area of the bottom of the lower lung lobe. Preferably, the area of the prosthesis projected to the bottom of the lower lung lobe will account for at least half of the area of the bottom of the lower lung lobe. Preferably, the area of the prosthesis projected to the bottom of the lower lung lobe will occupy at least 3/4 of the area of the bottom of the lower lung lobe. Preferably, the area of the prosthesis projected to the bottom of the lower lung lobe will account for the whole area of the bottom of the lower lung lobe.

The prosthesis in the present invention comprises a body having an upper side, a lower side and a lateral side extending between the upper side and the lower side. The prosthesis upper side is preferably matched with the bottom of the lobes. The prosthesis lower side is preferably matched with the diaphragm. The prosthesis outer lateral side is preferably matched with the wall of pleural cavity between the bottom of the lobes and the diaphragm far from the heart. The prosthesis inner lateral side is preferably matched with the outer wall of the lower lung lobe. The prosthesis is in a shape of plate, ellipse, irregular U, arc, conical, scapula, or irregular, and so on, so as to occupy the space under the lung lobes. The prosthesis has a smooth curved profile without edges and corners so as to reduce the discomfort and avoid injury to the pleura.

Alternatively, the prosthesis in the present invention comprises a body having an upper side, a lower side, a lateral side extending between the upper side and the lower side, and a medial border approaching the heart, wherein the lateral side is opposite to the medial border. The prosthesis upper side is preferably matched with the bottom of the lobes. The prosthesis lower side is preferably matched with the diaphragm. The prosthesis outer lateral side is preferably matched with the wall of pleural cavity between the bottom of the lobes and the diaphragm far from the heart. The prosthesis is in a shape of plate, ellipse, irregular U, arc, conical, scapula, or irregular, and so on, so as to occupy the space under the lower lung lobes. Preferably, the prosthesis upper side tapers toward the medial border. The prosthesis lower side tapers toward the medial border. The prosthesis has a smooth curved profile without edges and corners so as to reduce the discomfort and avoid injury to the pleura.

Preferably, the lateral side is at a height of 0.5cm-8cm. Preferably, the lateral side is at a height of 0.5cm, 0.6cm, 0.7cm, 0.8cm, 0.8cm, 0.9cm, 1cm, 2cm, 3cm, 4cm, 5cm, 6cm, 7cm, or 8cm.

Preferably, the thickness of the wall of the prosthesis is between 0.1-4cm. Preferably, the thickness is 0.1cm, 0.2cm, 0.3cm, 0.4cm, 0.5cm, 0.6cm, 0.7cm, 0.8cm, 0.9cm, 1cm, 1.1cm, 1.2cm, 1.3cm, 1.4cm, 1.5cm, 1.6cm, 1.7cm, 1.8cm, 1.9cm, 2cm, 3cm, or 4cm.

Preferably, the prosthesis in the present invention is in a shape of arc, wherein the arc is matched with the lower edge of the lower lung lobe. Preferably, the arch is matched with at least 1/8 of the lower edge of the lower lung lobe far from the heart. Preferably, the arch is matched with at least 1/4 of the lower edge of the lower lung lobe far from the heart. Preferably, the arch is matched with at least 1/2 of the lower edge of the lower lung lobe far from the heart. Preferably, the arch is matched with the overall length of the lower edge of the lower lung lobe.

Preferably, the prosthesis is at a height of 1cm-4cm. Preferably, the lateral side is at a height of 1cm, 2cm, 3cm, or 4cm.

Preferably, the prosthesis is designed to be placed in the pleural cavity, in particular, the prosthesis is placed at the lower side of the pleural cavity, so as to reduce the mechanical tension on the alveolar epithelium at the end of normal inspiratory. The prosthesis may be fixed in the pleural cavity, in particular, the prosthesis is placed at the lower side of the pleural cavity. For example, the prosthesis may be sutured to the pleural cavity wall using surgical sutures, particularly, the lower portion of the pleural cavity wall. Preferably, the prosthesis is sutured to of the wall of pleural cavity through the pinholes on the lateral side.

The prosthesis may be made of soft spongy latex, foam latex, 380 micron hollow fiber, gelatin foam, material, plastic sponge (Ivalon), polythene bag filled with fiberglass, rubber, silicone rubber, silicone gel, carbon materials involving carbon nanotube, grapheme, ultra-light porous carbon, hollow porous carbon, carbon fiber, carbon titanium alloy, and et.,al.

The prosthesis may be solid, or the prosthesis may be a pocket or a sack, filled with fluid or jelly.

In the second place, the invention provides use of prosthesis for treating pulmonary fibrosis, in particular, idiopathic pulmonary fibrosis (IPF).

The prosthesis in the present invention may be designed to be placed under the pulmonary lobe, as long as at the end of normal inspiratory, the level of mechanical tension on the alveolar epithelium is reduced.

Preferably, the prosthesis is designed to be anchored under the lobes and above the diaphragm, and preferably, is designed to match with the bottom of the lobes, as long as the mechanical tension on the alveolar epithelium is reduced at the end of normal inspiratory.

Therefore, the prosthesis is surrounded by pleural fluid. Preferably, the prosthesis will occupy at least 1/8 of the space surrounded by the bottom of the lobes, diaphragm and the wall of pleural cavity at the end of normal inspiratory. Preferably, the prosthesis will occupy at least 1/4 of the space surrounded by the bottom of the lobes, diaphragm and the wall of pleural cavity at the end of normal inspiratory. Preferably, the prosthesis will occupy at least half of the space surrounded by the bottom of the lobes, diaphragm and the wall of pleural cavity at the end of normal inspiratory. Preferably, the prosthesis will occupy at least 3/4 of the space surrounded by the bottom of the lobes, diaphragm and the wall of pleural cavity at the end of normal inspiratory. Preferably, the prosthesis will occupy the whole space surrounded by the bottom of the lobes, diaphragm and the wall of pleural cavity at the end of normal inspiratory.

Therefore, the prosthesis is surrounded by pleural fluid. Preferably, the area of the prosthesis projected to the bottom of the lower lung lobe will account for at least 1/8 of the area of the bottom of the lower lung lobe. Preferably, the area of the prosthesis projected to the bottom of the lower lung lobe will account for at least 1/4 of the area of the bottom of the lower lung lobe. Preferably, the area of the prosthesis projected to the bottom of the lower lung lobe will account for at least half of the area of the bottom of the lower lung lobe. Preferably, the area of the prosthesis projected to the bottom of the lower lung lobe will occupy at least 3/4 of the area of the bottom of the lower lung lobe. Preferably, the area of the prosthesis projected to the bottom of the lower lung lobe will account for the whole area of the bottom of the lower lung lobe.

The prosthesis in the present invention comprises a body having an upper side, a lower side and a lateral side extending between the upper side and the lower side. The prosthesis upper side is preferably matched with the basal of the lobes. The prosthesis lower side is preferably matched with the diaphragm. The prosthesis outer lateral side is preferably matched with the wall of pleural cavity between the bottom of the lobes and the diaphragm far away from the heart. The prosthesis inner lateral side is preferably matched with the outer wall of the lower lung lobe. The prosthesis may be in a shape of plate, ellipse, irregular U, arc, conical, scapula, or irregular, and so on, so as to occupy the space under the lung lobes. The prosthesis has a smooth curved profile without edges and corners so as to reduce the discomfort and avoid injury to the pleura.

Alternatively, the prosthesis in the present invention comprises a body having an upper side, a lower side, a lateral side extending between the upper side and the lower side, and a medial border approaching the heart, wherein the lateral side is opposite to the medial border. The prosthesis upper side is preferably matched with the bottom of the lobes. The prosthesis down side is preferably matched with the diaphragm. The prosthesis outer lateral side is preferably matched with the wall of pleural cavity between the bottom of the lobes and the diaphragm far away from the heart. The prosthesis is in a shape of plate, ellipse, irregular U, arc, conical, scapula, or irregular, and so on, so as to occupy the space under the lung lobes. Preferably, the prosthesis upper side tapers toward the medial border. The prosthesis lower side tapers toward the medial border. The prosthesis has a smooth curved profile without edges and corners so as to reduce the discomfort and avoid injury to the pleura.

Preferably, the lateral side is at a height of 0.5cm-8cm. Preferably, the lateral side is at a height of 0.5cm, 0.6cm, 0.7cm, 0.8cm, 0.8cm, 0.9cm, 1cm, 2cm, 3cm, 4cm, 5cm, 6cm, 7cm, or 8cm.

Preferably, the thickness of the wall of the prosthesis is between 0.1-4cm. Preferably, the thickness is 0.1cm, 0.2cm, 0.3cm, 0.4cm, 0.5cm, 0.6cm, 0.7cm, 0.8cm, 0.9cm, 1cm, 1.1cm, 1.2cm, 1.3cm, 1.4cm, 1.5cm, 1.6cm, 1.7cm, 1.8cm, 1.9cm, 2cm, 3cm, or 4cm.

Preferably, the prosthesis in the present invention is in a shape of arc, wherein the arc is matched with the lower edge of the lower lung lobe. Preferably, the arch is matched with at least 1/8 of the lower edge of the lower lung lobe far away from the heart. Preferably, the arch is matched with at least 1/4 of the lower edge of the lower lung lobe far from the heart. Preferably, the arch is matched with at least 1/2 of the lower edge of the lower lung lobe far away from the heart. Preferably, the arch is matched with the overall length of the lower edge of the lower lung lobe.

Preferably, the prosthesis is at a height of 1cm-4cm. Preferably, the lateral side is at a height of 1cm, 2cm, 3cm, or 4cm.

Preferably, the prosthesis is designed to be placed in the pleural cavity, in particular, the prosthesis is placed at the lower side of the pleural cavity, so as to reduce the mechanical tension on the alveolar epithelium at the end of normal inspiratory. The prosthesis may be fixed in the pleural cavity, in particular, the prosthesis is placed at the lower side of the pleural cavity. For example, the prosthesis may be sutured to the wall of pleural cavity, in particular, the lower part of wall of pleural cavity. Preferably, the prosthesis is sutured to the wall of pleural cavity through the pinholes on the lateral side.

The prosthesis may be made of soft spongy latex, foam latex, 380 micron hollow fiber, gelatin foam, material, plastic sponge (Ivalon), polythene bag filled with fiberglass, rubber, silicone rubber, silicone gel, carbon materials involving carbon nanotube, grapheme, ultra-light porous carbon, hollow porous carbon, carbon fiber, carbon titanium alloy, and et.,al.

The prosthesis may be solid, or the prosthesis may be a pocket or a sack, filled with fluid or jelly.

In the third place, the invention provides a method for treating pulmonary fibrosis, in particular, idiopathic pulmonary fibrosis (IPF), which involves a step of reducing the mechanical tension on the alveolar epithelium at the end of normal inspiratory. Preferably, the invention provides a method for treating pulmonary fibrosis, in particular, idiopathic pulmonary fibrosis (IPF), which involves placing prosthesis in the pleural cavity, in particular, the lower part of the pleural cavity, so as to reduce the level of mechanical tension on the alveolar epithelium at the end of normal inspiratory. The prosthesis may be fixed in the pleural cavity, in particular, the prosthesis is placed at the lower part of the pleural cavity. For example, the prosthesis may be sutured to the wall of pleural cavity, in particular, the lower part of the chest wall.

The prosthesis in the present invention may be designed to be placed under the pulmonary lobe, as long as at the end of normal inspiratory, the level of mechanical tension on the alveolar epithelium is reduced.

Preferably, the prosthesis is designed to be anchored under the lobes and above the diaphragm, and preferably, is designed to be matched with the basal of the lobes, as long as at the end of normal inspiratory, the level of mechanical tension on the alveolar epithelium is reduced.

Therefore, the prosthesis is surrounded by pleural fluid. Preferably, the prosthesis will occupy at least 1/8 of the space surrounded by the bottom of the lobes, diaphragm and the wall of pleural cavity at the end of normal inspiratory. Preferably, the prosthesis will occupy at least 1/4 of the space surrounded by the bottom of the lobes, diaphragm and the wall of pleural cavity at the end of normal inspiratory. Preferably, the prosthesis will occupy at least half of the space surrounded by the bottom of the lobes, diaphragm and the wall of pleural cavity at the end of normal inspiratory. Preferably, the prosthesis will occupy at least 3/4 of the space surrounded by the bottom of the lobes, diaphragm and the wall of pleural cavity at the end of normal inspiratory. Preferably, the prosthesis will occupy the whole space surrounded by the bottom of the lobes, diaphragm and the wall of pleural cavity at the end of normal inspiratory.

Therefore, the prosthesis is surrounded by pleural fluid. Preferably, the area of the prosthesis projected to the bottom of the lower lung lobe will account for at least 1/8 of the area of the bottom of the lower lung lobe. Preferably, the area of the prosthesis projected to the bottom of the lower lung lobe will account for at least 1/4 of the area of the bottom of the lower lung lobe. Preferably, the area of the prosthesis projected to the bottom of the lower lung lobe will account for at least half of the area of the bottom of the lower lung lobe. Preferably, the area of the prosthesis projected to the bottom of the lower lung lobe will occupy at least 3/4 of the area of the bottom of the lower lung lobe. Preferably, the area of the prosthesis projected to the bottom of the lower lung lobe will account for the whole area of the bottom of the lower lung lobe.

The prosthesis in the present invention comprises a body having an upper side, a lower side and a lateral side extending between the upper side and the lower side. The prosthesis upper side is preferably matched with the bottom of the lobes. The prosthesis down side is preferably matched with the diaphragm. The prosthesis outer lateral side is preferably matched with the wall of pleural cavity between the bottom of the lobes and the diaphragm far away from the heart. The prosthesis inner lateral side is preferably matched with the out wall of the lower lung lobe. The prosthesis may be in a shape of plate, ellipse, irregular U, arc, conical, scapula, or irregular, and so on, so as to occupy the space under the lung lobes. The prosthesis has a smooth curved profile without edges and corners so as to reduce the discomfort and avoid injury to the pleura.

Alternatively, the prosthesis in the present invention comprises a body having an upper side, a lower side, a lateral side extending between the upper side and the lower side, and a medial border approaching the heart, wherein the lateral side is opposite to the medial border. The prosthesis upper side is preferably matched with the basal of the lobes. The prosthesis lower side is preferably matched with the diaphragm. The prosthesis outer lateral side is preferably matched with the wall of pleural cavity between the bottom of the lobes and the diaphragm far from the heart. The prosthesis is in a shape of plate, ellipse, irregular U, arc, conical, scapula, or irregular, and so on, so as to occupy the space under the lung lobes. Preferably, the prosthesis upper side tapers toward the medial border. The prosthesis lower side tapers toward the medial border. The prosthesis has a smooth curved profile without edges and corners so as to reduce the discomfort and avoid injury to the pleura.

Preferably, the lateral side is at a height of 0.5cm-8cm. Preferably, the lateral side is at a height of 0.5cm, 0.6cm, 0.7cm, 0.8cm, 0.8cm, 0.9cm, 1cm, 2cm, 3cm, 4cm, 5cm, 6cm, 7cm, or 8cm.

Preferably, the thickness of the wall of the prosthesis is between 0.1-4cm. Preferably, the thickness is 0.1cm, 0.2cm, 0.3cm, 0.4cm, 0.5cm, 0.6cm, 0.7cm, 0.8cm, 0.9cm, 1cm, 1.1cm, 1.2cm, 1.3cm, 1.4cm, 1.5cm, 1.6cm, 1.7cm, 1.8cm, 1.9cm, 2cm, 3cm, or 4cm..

Preferably, the prosthesis in the present invention is in a shape of arc, wherein the arc is matched with the lower edge of the lower lung lobe. Preferably, the arch is matched with at least 1/8 of the lower edge of the lower lung lobe far away from the heart. Preferably, the arch is matched with at least 1/4 of the lower edge of the lower lung lobe far away from the heart. Preferably, the arch is matched with at least 1/2 of the lower edge of the lower lung lobe far away from the heart. Preferably, the arch is matched with the overall length of the lower edge of the lower lung lobe.

Preferably, the prosthesis is at a height of 1cm-4cm. Preferably, the lateral side is at a height of 1cm, 2cm, 3cm, or 4cm.

Preferably, the prosthesis is designed to be placed in the pleural cavity, in particular, the prosthesis is placed at the lower side of the pleural cavity, so as to reduce the mechanical tension on the alveolar epithelium at the end of normal inspiratory. The prosthesis may be fixed in the pleural cavity, in particular, the prosthesis is placedat the lower side of the pleural cavity. For example, the prosthesis may be sutured to the pleural cavity wall, in particular, the lower part of the pleural cavity wall. Preferably, the prosthesis is sutured to the wall of pleural cavity through the pinholes on the lateral side.

The prosthesis may be made of soft spongy latex, foam latex, 380 micron hollow fiber, gelatin foam, material, plastic sponge (Ivalon), polythene bag filled with fiberglass, rubber, silicone rubber, silicone gel, carbon materials involving carbon nanotube, grapheme, ultra-light porous carbon, hollow porous carbon, carbon fiber, carbon titanium alloy, and et.,al.

The prosthesis may be solid, or the prosthesis may be a pocket or a sack, filled with fluid or jelly.

The invention encompasses all combinations of the particular embodiments recited herein.

The invention encompasses all combination of the particular embodiments recited herein. Idiopathic pulmonary fibrosis (IPF) is a fatal progressive lung disease with few treatment options. Our invention demonstrates that impaired alveolar regeneration results in elevated mechanical tension that in turn drives the initiation and progression of lung fibrosis. We establish that prosthesis implantation in the pleural cavity, a simple therapeutic approach, greatly attenuates the progression of lung fibrosis. We anticipate that prosthesis implantation will lead to major breakthroughs to advance the future development of treatments for IPF. Therefore, the present invention is a pioneering invention, which achieves the treatment of IPF, a difficult disease in the world.

### Brief Description of the Drawings

Figures 1A-1F show six perspectives of a prosthesis manufactured in accordance with the present invention.
Figures 2A-2D show four perspectives of a prosthesis manufactured in accordance with the present invention.
Figures 3A-3D show four perspectives of a prosthesis manufactured in accordance with the present invention.
Figures 4A-4F show six perspectives of a prosthesis manufactured in accordance with the present invention.
Figures 5A-5E show five perspectives of a prosthesis manufactured in accordance with the present invention.
Figures 6A-6F show six perspectives of a prosthesis manufactured in accordance with the present invention.
Figures 7A-7C show three perspectives of a prosthesis manufactured in accordance with the present invention.
Figures 8A-8C show three perspectives of a prosthesis manufactured in accordance with the present invention.
Figures 9A-9C show three perspectives of a prosthesis manufactured in accordance with the present invention.
Figures 10A-10C show three perspectives of a prosthesis manufactured in accordance with the present invention.
Figures 11A-11C show three perspectives of a prosthesis manufactured in accordance with the present invention.
Figures 12A-12C show three perspectives of a prosthesis manufactured in accordance with the present invention.
Figures 13A-13D show four perspectives of a prosthesis manufactured in accordance with the present invention.
Figures 14A-14D show four perspectives of a prosthesis manufactured in accordance with the present invention.
Figures 15A-15D show four perspectives of a prosthesis manufactured in accordance with the present invention.
Figures 16A-16E show four perspectives of a prosthesis manufactured in accordance with the present invention.
Figure 17 illustrates a prosthesis of the present invention in position in a person's chest.
Figure 18 shows that a mouse line in which *Cdc42* gene is specifically deleted in AT2 cells is generated.
Figure 19 shows that loss of *Cdc42* gene in AT2 cells impairs the differentiation of AT2 cells during post-PNX alveolar regeneration or alveolar homeostasis.
Figure 20 shows that loss of *Cdc42* gene in AT2 cells leads to progressive lung fibrosis in PNX-treated mice.
Figure 21 shows that loss of *Cdc42* gene in AT2 cells leads to progressive lung fibrosis in non-PNX-treated aged mice.
Figure 22 shows the development of α-SMA⁺ fibroblastic foci in the lungs of *Cdc42* AT2 null mice.
Figure 23 shows that the expression level of CDC42-GTP increases significantly at post-PNX day 7.
Figure 24 shows that reducing mechanical tension on alveoli attenuates progressive lung fibrosis.
Figure 25 shows the fragments of *Cdc42* DNA sequence before or after deleting the exon2 of the *Cdc42* gene.

### Description of Particular Embodiments of the Invention

The descriptions of particular embodiments and examples are provided by way of illustration and not by way of limitation. Those skilled in the art will readily recognize a variety of noncritical parameters that could be changed or modified to yield essentially similar results.

The pleural cavity is the thin fluid-filled space between the two pulmonary pleurae (known as visceral and parietal) of each lung. A pleura is a serous membrane which folds back onto itself to form a two-layered membranous pleural sac. The outer pleura (parietal pleura) is attached to the chest wall, but is separated from it by the endothoracic fascia. The inner pleura (visceral pleura) covers the lung and adjacent structures, including blood vessels, bronchi and nerves. The pleural cavity can be viewed as a potential space because the two pleurae adhere to each other (through the thin film of serous liquid) under all normal conditions. The pleural cavity is surrounded by the rib cage, and it surrounds the lungs. A small amount of fluid is located in the potential space between the two layers of the pleura.

Pleural fluid is serous fluid produced by the serous membrane covering normal pleurae. Most fluid is produced by the parietal circulation (intercostal arteries) via bulk flow and reabsorbed by the lymphatic system. Thus, pleural fluid is produced and reabsorbed continuously.

The lungs are located in the chest on both sides of the heart in the rib cage. They are conical in shape with a narrow rounded apex at the top, and a broad concave base that rests on the convex surface of the diaphragm. The lungs are surrounded by the pulmonary pleurae. The pleurae are two serous membranes; the outer parietal pleura lines on the inner wall of the rib cage and the inner visceral pleura directly lines on the surface of the lungs. Between the pleurae is a potential space called the pleural cavity, which contains a thin layer of lubricating pleural fluid. Each lung is divided into lobes by the infoldings of the pleura as fissures. The fissures are double folds of pleura that separates the lungs and helps the lung to expand. The right lung has three lobes, namely, the superior (upper), middle and inferior (lower) lobes, and there are only two lobes in the left lung: the superior (upper) and inferior (lower) lung lobes.

The term "mechanical tension" in the present invention means "physical tension", "stretch", "distension", "stress", or "strain", for example, "strain" involves "compressive strain", "tensile strain", and an angular distortion is a "shear strain".

The idiopathic pulmonary fibrosis (IPF) is a type of chronic lung disease characterized by progressive and irreversible decline in lung function. Symptoms typically include gradual shortness of breath and a dry cough. Other changes may include feeling tired and nail clubbing. Complications may include pulmonary hypertension, heart failure, pneumonia, or pulmonary embolism.

Prosthesis is an artificial device that replaces a body part and is intended to restore the normal functions of the body part. Prostheses can be made by hand or with CAD (Computer-Aided Design), a software interface that helps creators to visualize the creation in a 3D form. The prosthetics are made in lightweight to facilitate the subject, and some of the materials include: plastics (polyethylene, polypropylene, acrylics, polyurethane), lightweight metals (titanium, aluminum), composite (carbon fibre),and silicone rubber.

Surgical suture is used to hold body tissues together after an injury or surgery. Surgical suture is made from numerous materials. The original suture is made from biological materials, such as catgut suture and silk, and synthetic materials, such as absorbable polyglycolic acid, polylactic acid, monocryl and polydioxanone as well as the non-absorbable nylon, polyester, PVDF and polypropylene. The polymer materials are based on one or more of five cyclic monomers: glycolide, l-lactide, p-dioxanone, trimethylene carbonate and ε-caprolactone.

Silicone rubber is an elastomer composed of silicone, a polymer, containing silicon together with carbon, hydrogen, and oxygen. Silicone rubber offers properties such as good resistance to extreme temperatures, elongation, creep, cyclic flexing, tear strength, compression set, dielectric strength (at high voltage), thermal conductivity, fire resistance and in some cases tensile strength can be-at extreme temperatures-far superior to organic rubbers in general.

The persons skilled in the art will anticipate that any drugs or prosthesis that reducing the mechanical tension of alveolar epithelium will be involved in the scope of the invention, since the inventors of the present invention firstly establish a direct linkage between the mechanical tension and the IPF, that is to say, reducing the mechanical tension of alveolar epithelium will be effective.

### Examples

### METHODS

### Mice and survival curve record.

*Rosa26*-CAG-mTmG (*Rosa26*-mTmG)²⁰ and *Cdc42* ^{flox/flox} mice²¹ have been described previously. All experiments were performed in accordance with the recommendations in the Guide for Care and Use of Laboratory Animals of the National Institute of Biological Sciences. To monitor the survival of mice, both the Control and the *Cdc42* AT2 null mice were weighed every week after the PNX treatment. Once the mice reached the pre-defined criteria for endpoints, the mice were sacrificed. We define the endpoints according to the pre-defined criteria^{22,23}.

### Generating Spc-CreER knock-in allele.

The CreERT2, p2a, and rtTA element were enzyme-linked and inserted into the mouse endogenous *Sftpc* gene. The insertion site is the stop codon of the endogenous *Sftpc* gene, then a new stop codon was created at the 3' end of rtTA. The CRISPR/Cas9 technology was used to insert the CreERT2-p2a-rtTA fragment into the genome.

### Pneumonectomy (PNX) and prosthesis implantation.

The male mice of 8 weeks old were injected with tamoxifen (dosage: 75mg/kg) every other day for 4 times. The mice were anesthetized and connected to a ventilator (Kent Scientific, Topo) from 14th day after the final dose of tamoxifen injection. The chest wall was incised at the fourth intercostal ribs and the left lung lobe was removed. For prosthesis implantation, a soft silicone prosthesis with a similar size and shape of the left lung lobe was inserted into the empty left lung cavity.

### Pulmonary function test.

Lung function parameters were measured using the invasive pulmonary function testing system (DSI Buxco^{®} PFT Controller). Mice were first anesthetized before inserting an endotracheal cannula into their trachea. The dynamic compliance results were obtained from the Resistance & Compliance Test.

### Hematoxylin and Eosin (H&E) staining and immunostaining.

Lungs were inflated with 4% paraformaldehyde (PFA) and were continualy fixed in 4% PFA at 4°C for 24 hours. Then the lungs were cryoprotected in 30% sucrose and embedded in OCT (Tissue Tek).

The H&E staining experiment followed the standard H&E protocol. Briefly, slides were washed by water to remove the OCT. The nuclei were stained by hemotoxylin (Abcam, ab150678) for 2 minutes and the cytoplasm was stained by eosin (Sigma, HT110280) for 3 minutes. Slices were sealed with neutral resin after the dehydration and clearing steps.

The immunofluorescence staining experiments followed the protocol previously described²⁴. In brief, after removing the OCT, the lung slices were blocked with 3%BSA/0.1%TritonX-100/PBS for 1 hour, then slides were incubated with primary antibodies at 4°C for overnight. After washing the slides with 0.1%TritonX-100/PBS for 3 times, the slices were incubated with secondary antibodies for 2 hours at room temperature.

The primary antibodies used in the paper are listed below:

| Name | Company and catalog number | Dilution |
|---|---|---|
| Chicken anti-GFP | Abcam, ab13970-100 | 1:500 |
| Rabbit anti-Collagen I | Abcam, ab34710 | 1:300 |
| Mouse anti α-SMA | Sigma, C6198 | 1:300 |
| Hamster anti-Pdpn | Developmental Studies Hybridoma Bank, clone8.1.1 | 1:100 |
| Rat anti-Ki67 | Bioscience, 514-5698-82 | 1:300 |

The secondary antibodies used in the paper are listed below:

| Name | Company and catalog number | Dilution |
|---|---|---|
| Alexa Fluor 488 Donkey anti-Chicken | 703-545-155, Jackson Immuno Research | 1:500 |
| Alexa Fluor 488 Donkey anti-mouse | 715-545-150, Jackson Immuno Research | 1:500 |
| Alexa Fluor 568 Donkey anti-rabbit | A11057, Invitrogen | 1:500 |
| Alexa Fluor 647 Goat anti-hamster | A-21451, Invitrogen | 1:500 |
| Biotin Donkey Anti-Rabbit | 711-065-152, Jackson Immuno Research | |

**Statistical analysis.** All data are presented as mean ± s.e.m. (as indicated in figure legends). The data presented in the figures were collected from multiple independent experiments that were performed on different days using different mice. Unless otherwise mentioned, most of the data presented in figure panels are based on at least three independent experiments. The inferential statistical significance of differences between sample means was evaluated using two-tailed unpaired Student's t-tests.

**Isolating mouse AT2 cells.** After 4 doses of tamoxifen injection, the lungs of *Spc-*CreER, *Rosa26*-mTmG mice were dissociated as previously described^{19,44}. Briefly, anesthetized mice were inflated with neutral protease (Worthington-Biochem, LS02111) and DNase I (Roche, 10104159001). AT2 cells were directly sorted based on the GFP fluorescence using the single-cell-select-mode in BD FACS Aria II and III appliances.

**Quantitative RT-PCR (qPCR).** Total RNA was isolated from either whole lung or primary AT2 cells using Zymo Research RNA Mini Prep Kits (R2050). Reverse transcription reactions were performed with a two-step cDNA synthesis Kit (Takara, Cat. # 6210A/B) according to the manufacturer's recommendations. qPCR was done with a CFX96 Touch^{™} Real-Time PCR Detection System. The mRNA levels of target genes were normalized to the *Gapdh* mRNA level.

Primers used for qPCR are listed below.

| | Forward | Reverse |
|---|---|---|
| *Gapdh* | AAGGTCGGTGTGAACGGATTTGG(S EQ ID NO:1) | CGTTGAATTTGCCGTGAGTGGAG( SEQ ID NO:2) |
| *Sftpc* | TTGTCGTGGTGATTGTAGGG(SEQ ID NO:3) | TGGAAAAGGTAGCGATGGTG(SEQ ID NO:4) |
| *Scd1* | GCAAGCTCTACACCTGCCTCTT(SEQ ID NO:5) | CGTGCCTTGTAAGTTCTGTGGC(SE Q ID NO:6) |
| *Lyz2* | TGCCAGAACTCTGAAAAGGAATGG( SEQ ID NO:7) | CAGTGCTTTGGTCTCCACGGTT(SE Q ID NO:8) |
| *Cbr2* | CATGGGCAAGAAAGTCTCTGCAG(S EQ ID NO:9) | ACTGGTAGAGGCACTTCTGTCG(SE Q ID NO:10) |
| *Sftpa1* | ACCTGGATGAGGAGCTTCAGAC(SE Q ID NO:11) | CTGACTGCCCATTGGTGGAAAAG( SEQ ID NO:12) |
| *Actb* | CATTGCTGACAGGATGCAGAAGG(S EQ ID NO:13) | TGCTGGAAGGTGGACAGTGAGG(S EQ ID NO:14) |
| *Actn1* | TCGCCAAGTGTCAACGCTCGTT(SEQ ID NO:15) | GGTCGATGGTTTCCAGCAGCTT(SE Q ID NO:16) |
| *Pfn1* | CATCGTAGGCTACAAGGACTCG(SE Q ID NO:17) | CCAAGTGTCAGCCCATTGACGA(S EQ ID NO:18) |
| *Ezr* | ATCGAGGTGCAGCAGATGAAGG(SE Q ID NO:19) | CGGAGCATCTGCTCCTTTTCTC(SE Q ID NO:120) |
| *Gsn* | GGCTTTGAGTCGTCCACCTTCT(SEQ ID NO:21) | GTCCTTTGACCTGGAAGAGCCT(SE Q ID NO:22) |

**3D alveolar reconstruction.** For vibratome sections, lungs were gently inflated to full capacity with 2% low-melting agarose. Then lungs were fixed in 4% PFA for overnight at 4°C. Thick vibratome sections were sliced at a thicknesses of 200µm using the vibrating microtome (Leica VT100S). Immunostaining experiments were performed as the standard wholemount staining protocol. Z stack images were taken by Leica LSI macro confocal microscope and/or A1-R inverted confocal microscope.

**CDC42-GTP assay.** The GTP-CDC42 level is determined using the CDC42 activation assay biochem kit (cytoskeleton, #BK127) according to the provided manufacturer's recommendations. Briefly, the whole lung lobes were grinded in liquid nitrogen, then lysed using the cell lysis buffer (applied in the kit). Then the cell lysates were added into the microplate wells applied. After the reaction, the absorbance at 490nm was measured.

### Example 1

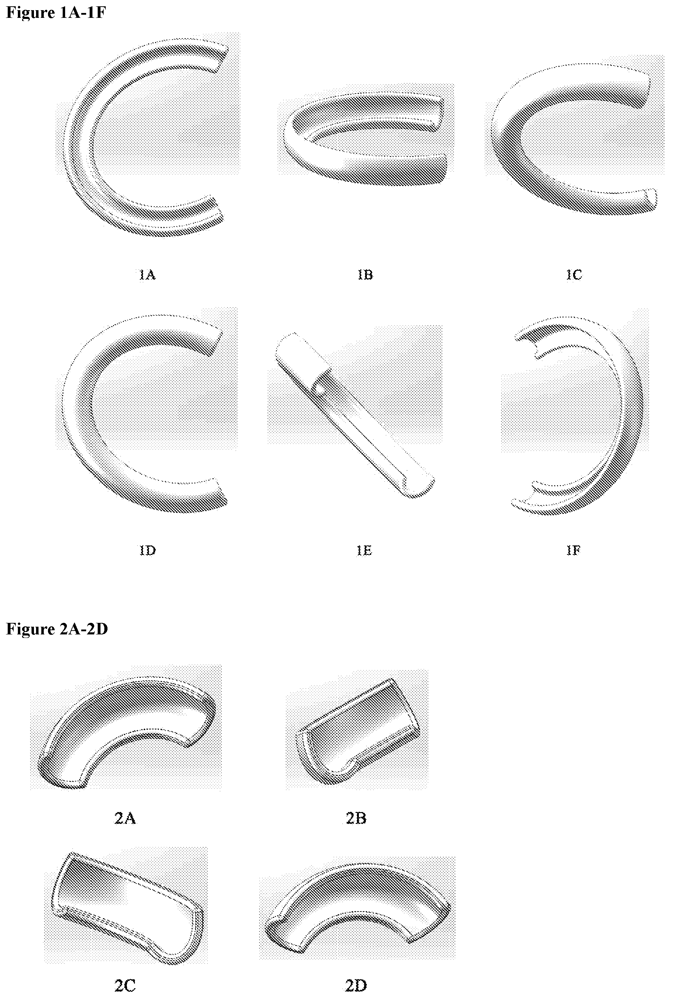

As shown in Figures 1A-1F, the prosthesis is in a shape of arc and occupies about 3/4 of the edge of the lower part of the lung lobe. The lateral side has a height of about 1.5cm. The prosthesis has a thickness of about 2mm. The bottom of the prosthesis has a width of 1cm. The prosthesis may be sewed onto the wall of pleural cavity. The sides of the prosthesis are round and smooth as far as possible.

The opening is toward the heart. At the end of normal inspiratory, the prosthesis presses the lower part of the lung lobe and the mechanical tension of alveolar epithelium is reduced.

### Example 2

As shown in Figures 2A-2D, the prosthesis is in a shape of arc and occupies about 1/8 of the edge of the lower part of the lung lobe. The lateral side has a height of about 3cm. The prosthesis has a thickness of about 7mm. The bottom of the prosthesis has a width of 2cm. The prosthesis may be sewed onto the side wall of pleural cavity far from the heart. The sides of the prosthesis are round and smooth as far as possible.

At the end of normal inspiratory, the prosthesis presses the lower part of the lung lobe and the mechanical tension of alveolar epithelium is reduced.

### Example 3

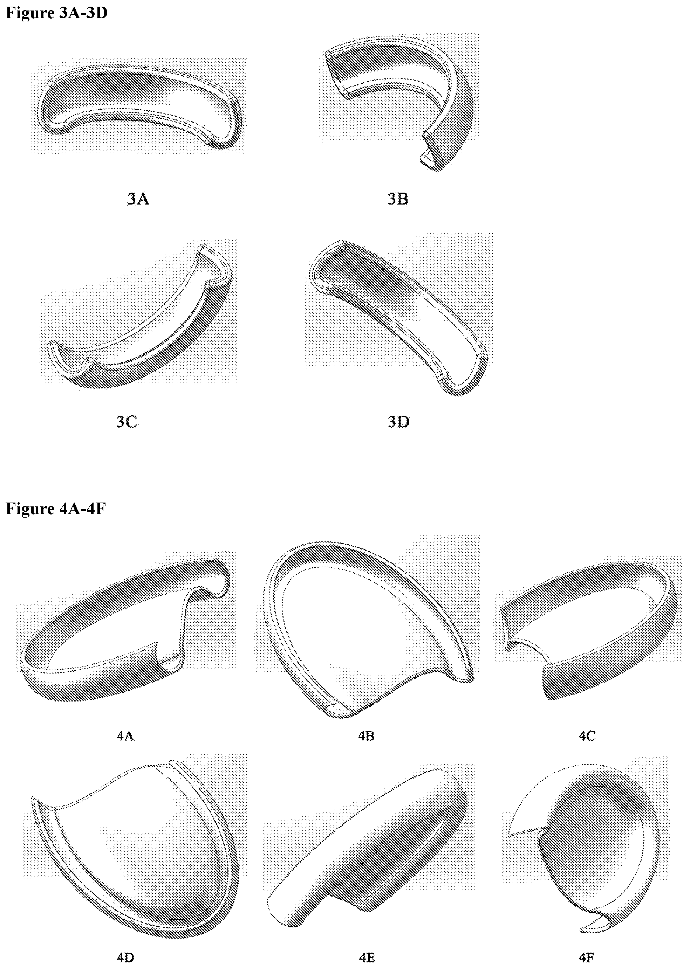

As shown in Figures 3A-3D, the prosthesis is in a shape of arc and occupies about 1/4 of the edge of the lower part of the lung lobe. The lateral side has a height of about 3cm. The prosthesis has a thickness of about 6mm. The bottom of the prosthesis has a width of 1.3cm. The prosthesis may be sewed onto the side wall of pleural cavity far from the heart. The sides of the prosthesis are round and smooth as far as possible.

At the end of normal inspiratory, the prosthesis presses the lower part of the lung lobe and the mechanical tension of alveolar epithelium is reduced.

### Example 4

As shown in Figures 4A-4F, the prosthesis is in a shape of plate and matches the whole area of the basal of the lower lung lobe. The lateral side has a height of about 2.5cm. The prosthesis has a thickness of about 2mm. The prosthesis may be sewed onto the side wall of pleural cavity. The sides of the prosthesis are round and smooth as far as possible.

At the end of normal inspiratory, the prosthesis presses the lower part of the lung lobe and the level of mechanical tension of alveolar epithelium is reduced.

### Example 5

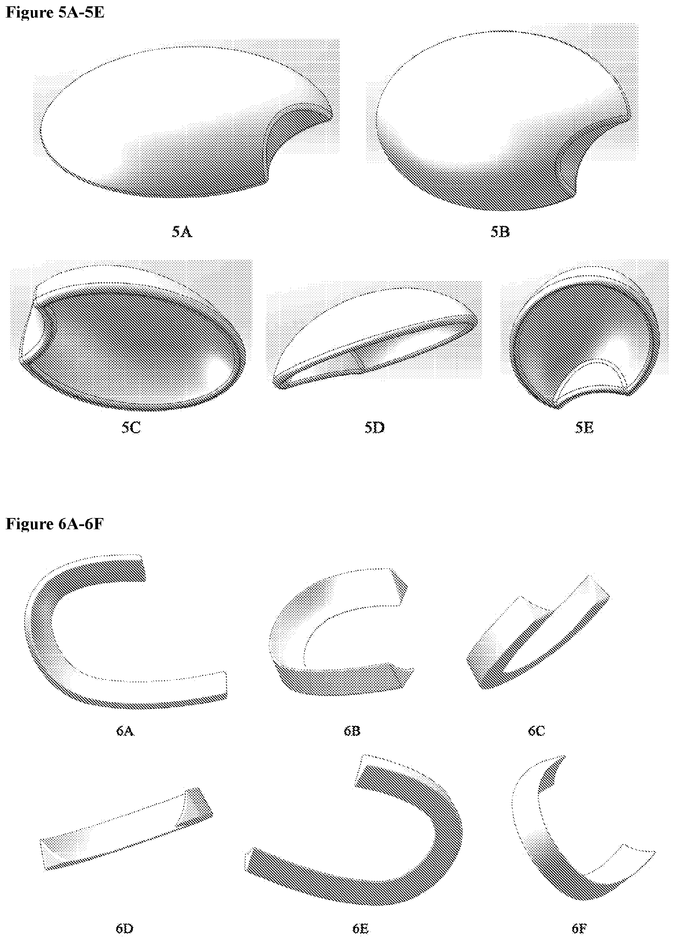

As shown in Figures 5A-5E, the prosthesis is in a shape of shell-like and matches the whole area of the bottom of the lower lung lobe. The prosthesis has depression in the middle and tapers to the side wall of pleural cavity. The prosthesis also has a depression on the side of heart to hold the heart. The prosthesis has a thickness of about 4mm. The sides of the prosthesis are round and smooth as far as possible.

At the end of normal inspiratory, the prosthesis presses the lower part of the lung lobe and the mechanical tension of alveolar epithelium is reduced.

### Example 6

As shown in Figures 6A-6F, the prosthesis is in a shape of U-like and matches more than 3/4 of the edge of the lower lung lobe. The section of the prosthesis is close to triangle. The side touching the edge of the lower lung lobe is a concave arc. The outer side of the prosthesis has height of 1.0cm. The sides of the prosthesis are round and smooth as far as possible.

The prosthesis may be sewed onto the side wall of pleural cavity. At the end of normal inspiratory, the prosthesis presses the lower part of the lung lobe and the mechanical tension of alveolar epithelium is reduced.

### Example 7

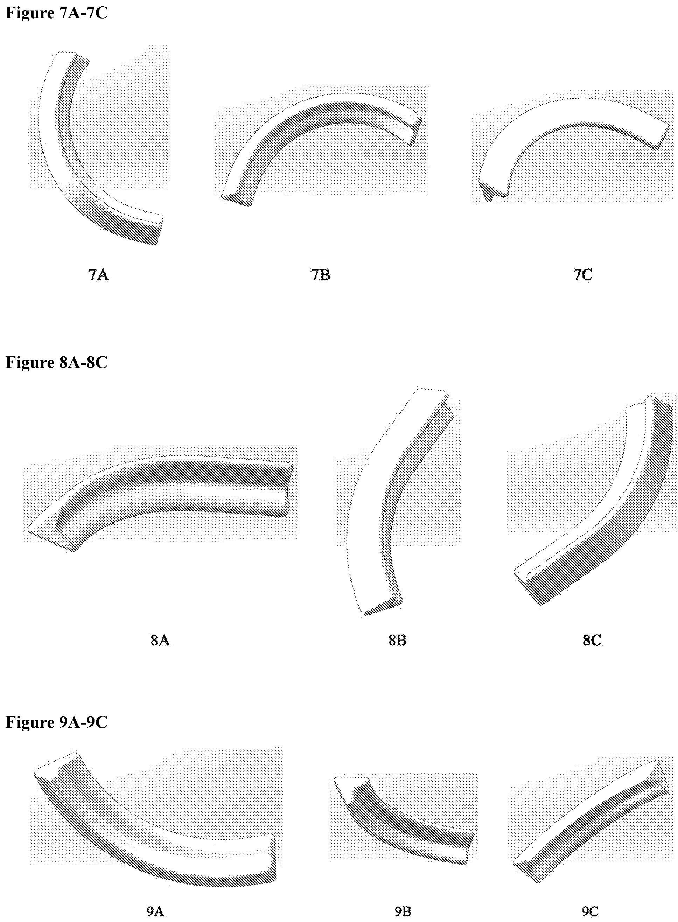

As shown in Figures 7A-7C, the prosthesis is in a shape of arc and matches more than 1/4 of the edge of the lower lung lobe. The section of the prosthesis is close to triangle. The side touching the edge of the lower lung lobe is a concave arc. The outer side of the prosthesis has a height of 1.6cm. The sides of the prosthesis are round and smooth as far as possible.

The prosthesis may be sewed onto the side wall of pleural cavity far from the heart. At the end of normal inspiratory, the prosthesis presses the lower part of the lung lobe and the mechanical tension of alveolar epithelium is reduced.

### Example 8

As shown in Figures 8A-8C, the prosthesis is in a shape of arc and matches more than 1/4 of the edge of the lower lung lobe. The prosthesis has a smaller radian than the prosthesis in Example 7. The section of the prosthesis is close to triangle. The side touching the edge of the lower lung lobe is a concave arc. The outer side of the prosthesis has a height of 2.2cm. The sides of the prosthesis are round and smooth as far as possible.

The prosthesis may be sewed onto the side wall of pleural cavity far from the heart. At the end of normal inspiratory, the prosthesis presses the lower part of the lung lobe and the mechanical tension of alveolar epithelium is reduced.

### Example 9

As shown in Figures 9A-9C, the prosthesis is in a shape of arc and matches more than 1/8 of the edge of the lower lung lobe. The prosthesis has a smaller radian than the prosthesis in Example 7. The section of the prosthesis is close to triangle. The side touching the edge of the lower lung lobe is a concave arc. The outer side of the prosthesis has a height of 3.5cm. The sides of the prosthesis are round and smooth as far as possible.

The prosthesis may be sewed onto the side wall of pleural cavity far from the heart. At the end of normal inspiratory, the prosthesis presses the lower part of the lung lobe and the mechanical tension of alveolar epithelium is reduced.

### Example 10

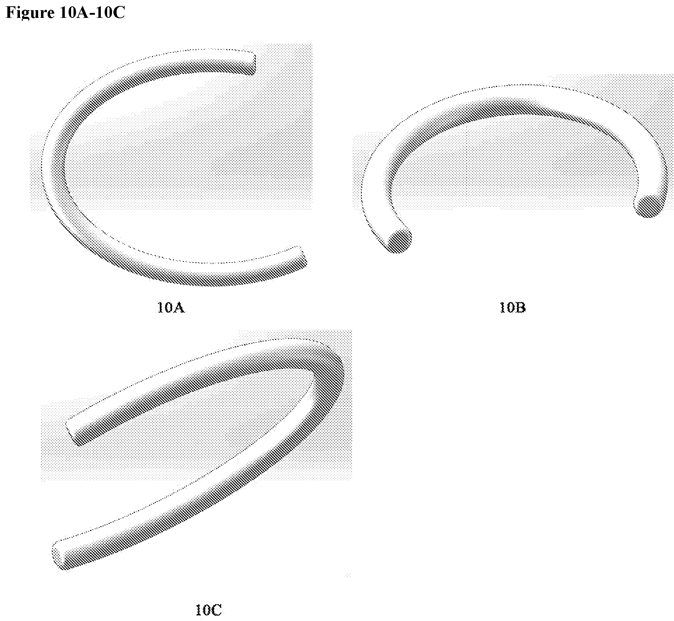

As shown in Figures 10A-10C, the prosthesis is in a shape of circular arc and matches more than 3/4 of the edge of the lower lung lobe. The section of the prosthesis is close to circular. The radius of the section is about 0.5cm.

At the end of normal inspiratory, the prosthesis presses the lower part of the lung lobe and the mechanical tension of alveolar epithelium is reduced.

### Example 11

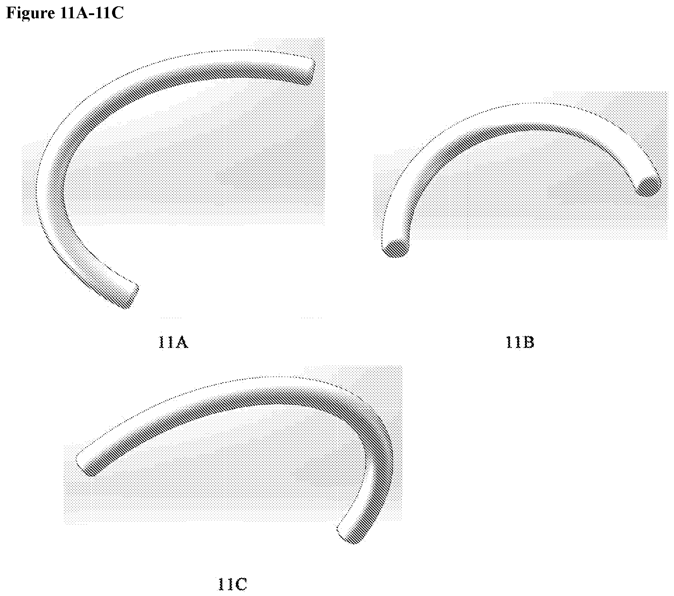

As shown in Figures 11A-11C, the prosthesis is in a shape of arc and matches more than 1/4 of the edge of the lower lung lobe. The section of the prosthesis is close to circular. The radius of the section is about 0.8cm.

The prosthesis may be sewed onto the side wall of pleural cavity far from the heart. At the end of normal inspiratory, the prosthesis presses the lower part of the lung lobe and the mechanical tension of alveolar epithelium is reduced.

### Example 12

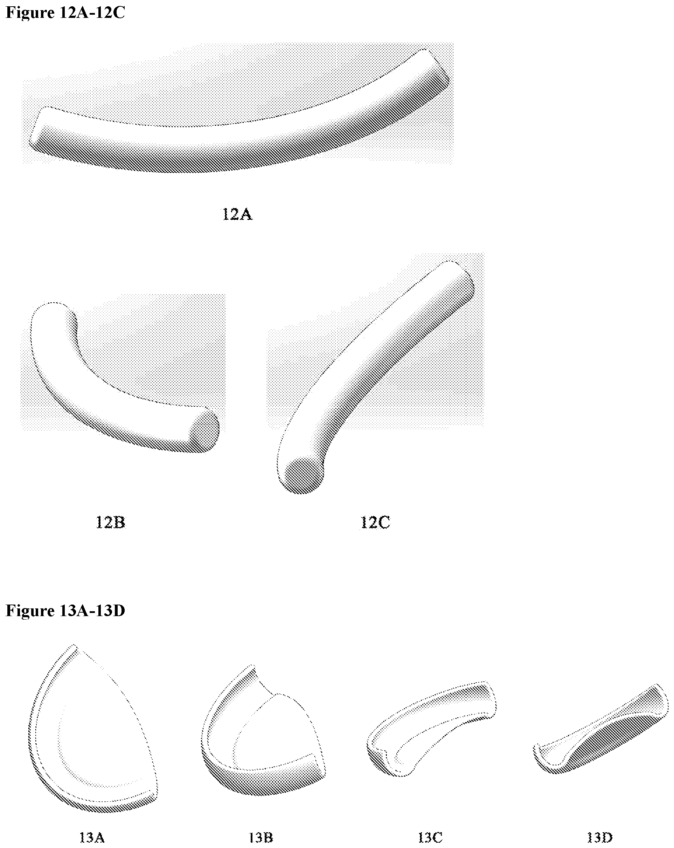

As shown in Figures 12A-12C, the prosthesis is in a shape of circular arc and matches more than 1/8 of the edge the lower lung lobe. The section of the prosthesis is close to circular. The radius of the section is about 1.0cm.

The prosthesis may be sewed onto the side wall of pleural cavity far from the heart. At the end of normal inspiratory, the prosthesis presses the lower part of the lung lobe and the mechanical tension of alveolar epithelium is reduced.

### Example 13

As shown in Figures 13A-13D, the prosthesis is in a shape of plate and matches more than 1/3 area of the basal of the lower lung lobe. The lateral side has a height of about 1.0cm. The prosthesis has a thickness of about 0.4cm. The prosthesis has a concave bottom. The prosthesis may be sewed onto the side wall of pleural cavity. The sides of the prosthesis are round and smooth as far as possible.

At the end of normal inspiratory, the prosthesis presses the lower part of the lung lobe and the mechanical tension of alveolar epithelium is reduced.

### Example 14

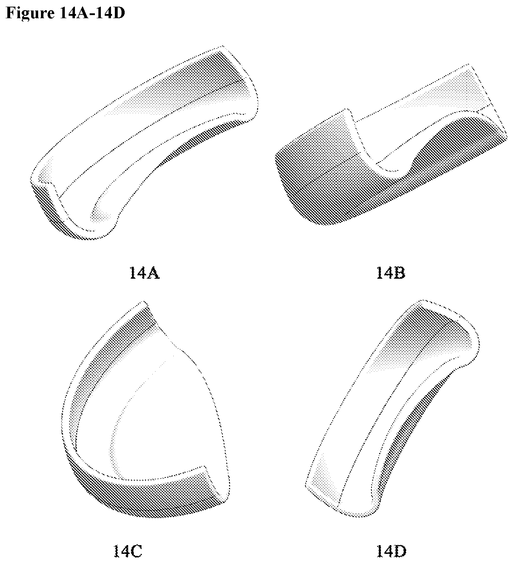

As shown in Figures 14A-14D, the prosthesis is in a shape of plate and matches more than 1/3 area of the basal of the lower lung lobe. The lateral side has a height of about 4cm. The prosthesis has a thickness of about 1.0cm. The prosthesis may be sewed onto the side wall of pleural cavity. The sides of the prosthesis are round and smooth as far as possible.

At the end of normal inspiratory, the prosthesis presses the lower part of the lung lobe and the mechanical tension of alveolar epithelium is reduced.

### Example 15

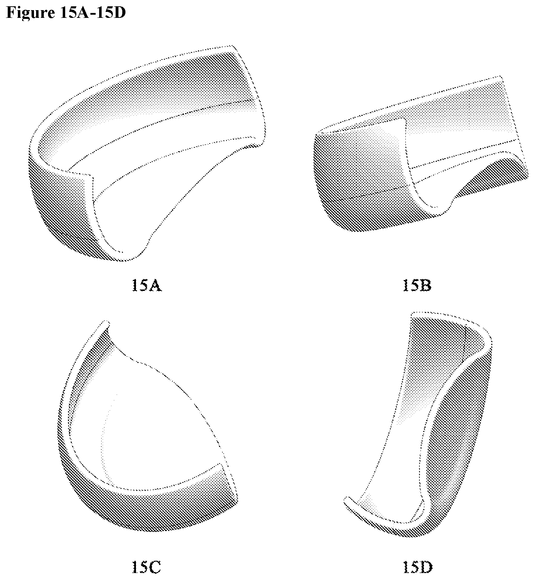

As shown in Figures 15A-15D, the prosthesis is in a shape of half plate and matches more than 1/3 area of the basal of the lower lung lobe. The lateral side has a height of about 5cm. The prosthesis has a thickness of about 0.5cm. The prosthesis may be sewed onto the side wall of pleural cavity. The sides of the prosthesis are round and smooth as far as possible.

At the end of normal inspiratory, the prosthesis presses the lower part of the lung lobe and the mechanical tension of alveolar epithelium is reduced.

### Example 16

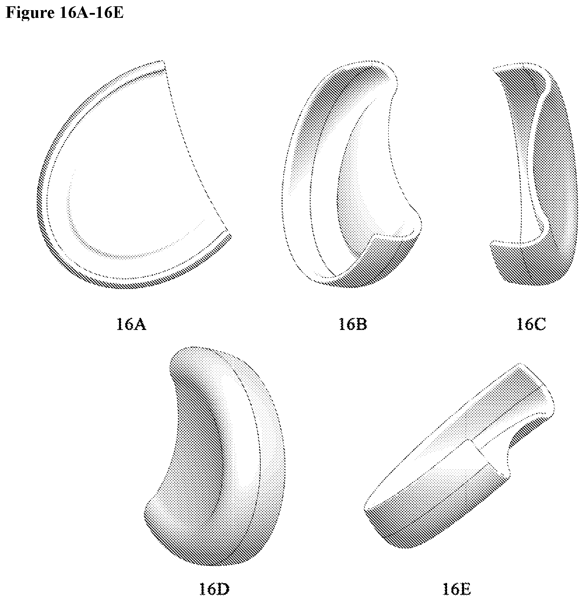

As shown in Figures 16A-16E, the prosthesis is in a shape of half plate and matches more than 1/3 area of the basal of the lower lung lobe. The lateral side has a height of about 3cm. The prosthesis has a thickness of about 0.3cm. The prosthesis may be sewed onto the side wall of pleural cavity. The sides of the prosthesis are round and smooth as far as possible.

At the end of normal inspiratory, the prosthesis presses the lower part of the lung lobe and the mechanical tension of alveolar epithelium is reduced.

### Example 17

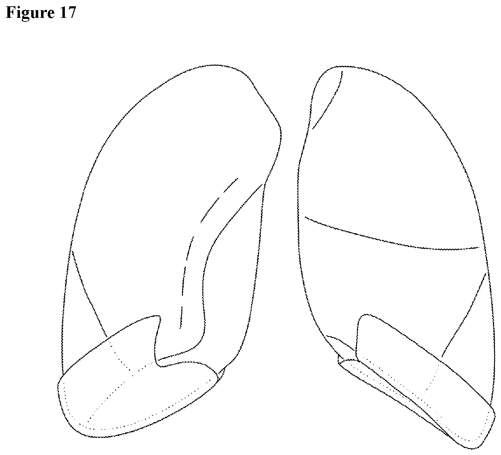

As shown in Figure 17, a prosthesis of Example 4 is positioned in a persons's chest. At the end of normal inspiratory, the prosthesis presses the lower part of the lung lobe and the mechanical tension of alveolar epithelium is reduced.

### Example 18

### Generation and characterization of Cdc42 AT2 null mice

In order to construct a progressive lung fibrosis animal model, *Cdc42* AT2 null mice are generated by knocking out *Cdc42* gene specifically in alveolar type II epithelial cells (AT2 cells).

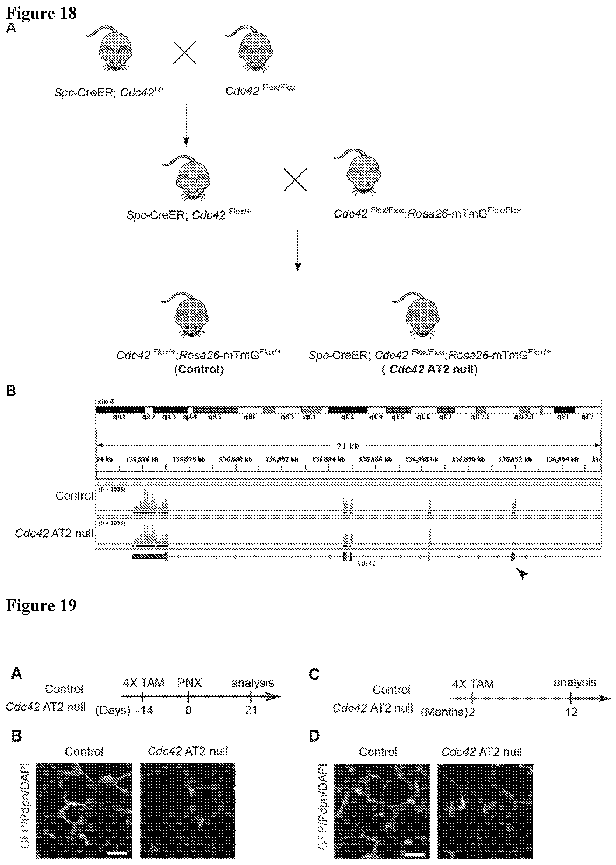

In order to specifically delete *Cdc42* gene in AT2 cells, mice carrying a *Spc*-CreER knock-in allele are crossed with the *Cdc42* floxed (*Cdc42*^{flox/flox}) mice (Figure18A). In *Cdc42* ^{flox/flox} mice, the exon 2 of *Cdc42* gene, which contains the translation initiation exon of *Cdc42* gene, is flanked by two loxp sites. In *Spe-CreER; Cdc42*^{flox/flox} mice, the exon 2 of *Cdc42* gene is specifically deleted in AT2 cells by Cre/loxp-mediated recombination after tamoxifen treatment (Figure 18B). *Spe-CreER; Cdc42*^{flox/flox} mice are named as *Cdc42* AT2 null mice.

The *Spc*-CreER, *Cdc42* ^{flox/-} mice were performed genome purification and PCR amplification. Then the flox and null bands of *Cdc42* were purified and sequenced using the primers as below: CTGCCAACCATGACAACCTAA (SEQ ID NO:23); AGACAAAACAACAAGGTCCAG (SEQ ID NO:24).

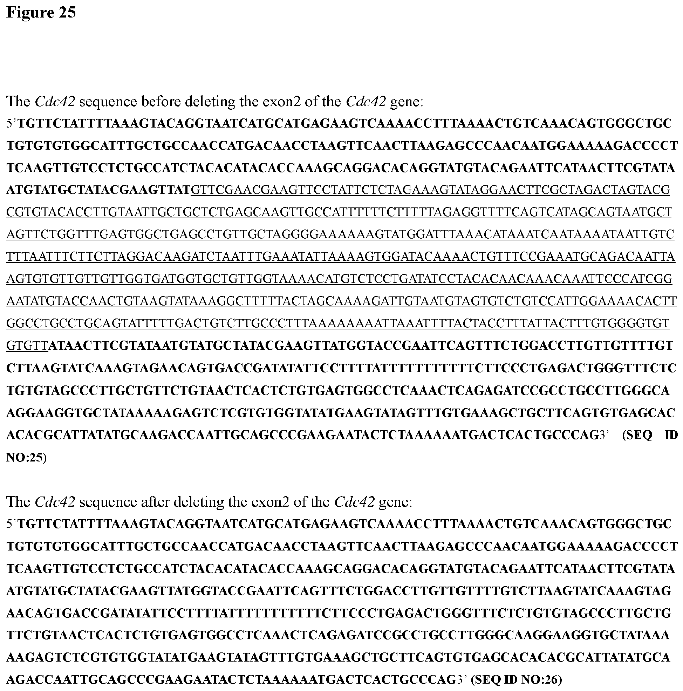

The fragments of *Cdc42* DNA sequence before or after deleting the exon2 of the *Cdc42* gene are shown in Figure 25.

As shown in (Figure 19A), 200µm lung sections of Control and *Cdc42* AT2 null mice are immunostained with antibodies against GFP, Pdpn, and Prospc. At post-PNX day 21, many newly differentiated AT1 cells and newly formed alveoli are observed in Control lungs (Figure 19B). However, in *Cdc42* AT2 null lungs, few AT2 cells have differentiated into AT1 cells, and no new alveoli are formed at post-PNX day 21 (Figure 19B). It is observed that the alveoli in peripheral region of PNX-treated *Cdc42* AT2 null lungs are profoundly overstretched (Figure 19B).

During alveolar homeostasis, AT2 cells slowly differentiate into AT1 cells and build new alveoli. When 12-month *Cdc42* AT2 null mice that had not undergone PNX are examined, enlarged alveoli with lacking any new AT1 cell formation are observed; and in contrast, the lungs of 12-month Control mice exhibit formation of many new alveoli (Figure 19C and 19D).

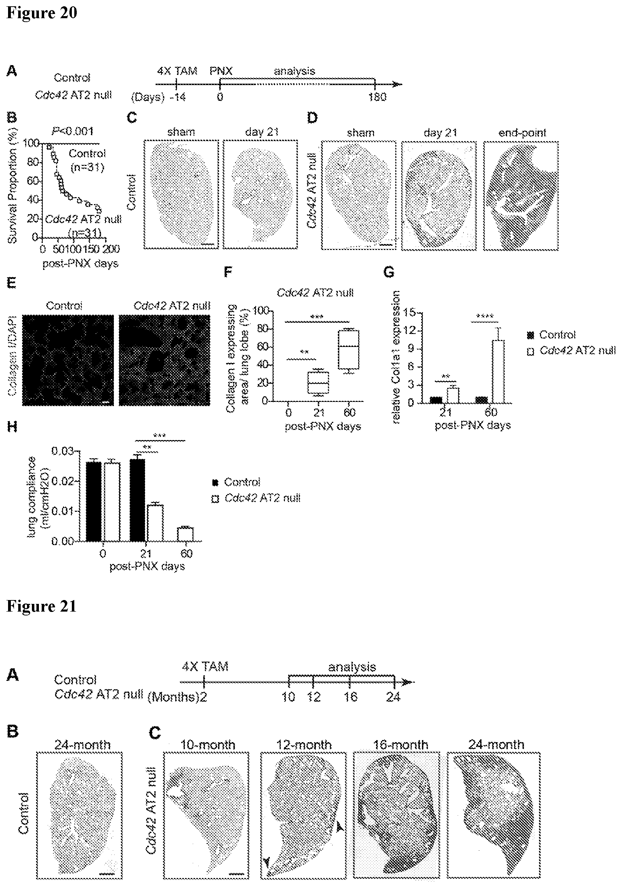

PNX-treated *Cdc42* AT2 null and Control mice are observed for a longer period of time after PNX treatment (Figure 20A). Some *Cdc42* AT2 null mice show significant weight loss and increased respiration rates at post-PNX day 30. Indeed, fully 50% of PNX-treated *Cdc42* AT2 null mice reach the predefined health-status criteria for endpoint euthanization by post-PNX day 60 (Figure 20B), and about 80% of PNX-treated *Cdc42* AT2 null mice reach their endpoints by post-PNX day 180 (Figure 20B).

H&E staining of PNX-treated Control and *Cdc42* AT2 null mice reveals severe fibrosis in the lungs of *Cdc42* AT2 null mice at their endpoints (Figures 20C-20D). In order to determine the point at which *Cdc42* AT2 null mice begin to develop lung fibrosis following PNX, the lungs of *Cdc42* AT2 null mice are analyzed at various time points after PNX using H&E staining (Figure 20D). The subpleural regions of some *Cdc42* AT2 null lungs exhibit signs of tissue thickening by post-PNX day 21 (Figure 20D compared with control lung in Figure 20C). By the end-point, the dense fibrosis has progressed to the centre of most *Cdc42* AT2 null lungs.

In addition to detecting strong immunofluorescence signals for Collagen I in these dense fibrotic regions (Figure 20E), we observe the proportion of Collagen I expressing area per lobe gradually increased in *Cdc42* AT2 null mice after PNX (Figure 20F). Our qPCR analysis also shows that the *Collagen I* mRNA expression levels increased gradually from post-PNX day 21 (Figure 20G). Moreover, gradually decreased lung compliance is observed in PNX-treated *Cdc42* AT2 null mice from post-PNX day 21 as compared to their PNX-treated Control mice (Figure 20H), an intriguing finding given that decreased lung compliance is known to occur frequently as lungs become fibrotic²⁰⁻²⁵.

Control and *Cdc42* AT2 null mice were exposed to 4 doses of tamoxifen 14 days starting at age of 2 months. Lungs of Control and *Cdc42* AT2 null mice without PNX treatment were collected at 10, 12, 16, or 24 months (Figure 21A). The lungs of Control and *Cdc42* AT2 null mice without PNX treatment were analyzed and found no significant fibrotic changes before the *Cdc42* AT2 null mice reached 10-months of age (Figures 21B and 21C). By 12 months, fibrosis had obviously begun to develop in the subpleural regions of *Cdc42* AT2 null lungs and to progress toward the center of the lung (Figure 21C). Thus, the loss of *Cdc42* in AT2 cells leads to progressive lung fibrosis in no-PNX-treated *Cdc42* AT2 null mice starting from around 12 months of age.

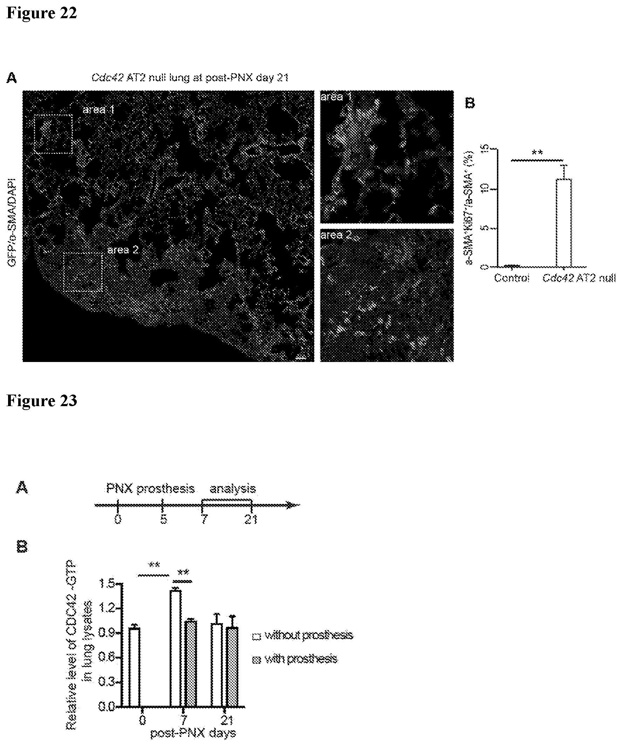

Fibroblastic foci are considered a relevant morphologic marker of progressive pulmonary fibrosis and are recognized as sites where fibrotic responses are initiated and/or perpetuated in progressive pulmonary fibrosis. The fibroblastic foci contain proliferating α-SMA⁺ fibroblasts. Lungs of *Cdc42* AT2 null mice at post-PNX day 21 are stained with antibodies against α-SMA (Figure 22A). Some α-SMA⁺ fibroblasts accumulating next to a cluster of AT2 cells (GFP⁺ cells) in the relative normal alveolar regions of *Cdc42* AT2 null lungs are observed (area 1, Figure 22A). And the dense fibrosis region of the lungs is filled with α-SMA⁺ fibroblasts (area 2, Figure 22A). In addition, the cell proliferation of α-SMA⁺ cells is increased dramatically in the lungs of *Cdc42* AT2 null mice at post-PNX day 21 by immunostaining using antibodies against both α-SMA and proliferation marker, Ki67, indicating that the proliferating α-SMA⁺ fibroblasts contribute to the development of lung fibrosis (Figure 22B).

### Example 19

### Elevated mechanical tension caused by impaired alveolar regeneration leads to progressive lung fibrosis

The fact that lung fibrosis in *Cdc42* AT2 null mice is greatly accelerated by the PNX treatment (Figure 20) suggests a close link between lung fibrosis and mechanical tension-induced alveolar regeneration.

The loss of alveoli resulting from PNX substantially increases mechanical tension exerted upon the alveolar epithelium. The subsequent efficient regeneration of alveoli that occurs in normal mice eventually reduces the intensity of the mechanical tension to pre-PNX levels; however, as *Cdc42* null AT2 cells are unable to differentiate into AT1 cells and thus cannot regenerate new alveoli (Figure 19B), the alveolar epithelium of *Cdc42* AT2 null mice continue to experience elevated mechanical tension (Figure 19B), which results in the progressive development of fibrosis(Figure 20D).

### Example 20

### Progressive lung fibrosis can be prevented by the prosthesis implantation

By measuring the expression levels of CDC42-GTP, the GTP-bound state of CDC42, in the post-PNX lungs, it is found that the activity of CDC42-GTP increased significantly at post-PNX day 7 (Fig. 23A-23B). Such increased expression of CDC42-GTP can be inhibited by implanting a prosthesis in the chest (Figures 23A-23B).

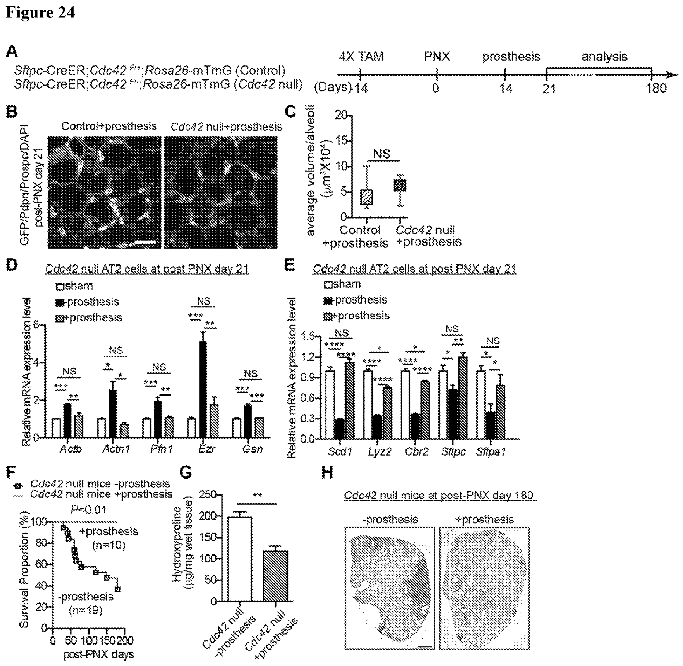

As shown in Figures 24A-24H, reducing mechanical tension on alveoli attenuate progressive lung fibrosis. Control and *Cdc42* AT2 null mice were exposed to 4 doses of tamoxifen 14 days prior to PNX. A prosthesis was implanted at post-PNX day 14 (Figure 24A). Lungs of prosthesis-implanted Control and *Cdc42* AT2 null mice were collected at post-PNX day 21. Images show the maximum intensity of a 200µm Z-projection of lung sections that were stained with antibodies against GFP, Pdpn, and Prospc (Figure 24B). The average alveolar size of prosthesis-implanted Control and *Cdc42* AT2 null mice is not significantly different at post-PNX day 21 (mean±S.E.M., n=3), indicating the enlarged alveoli phenotype of *Cdc42* AT2 null lungs can be greatly rescued by the implantation of the prosthesis (Figures 24B-24C). Recalling moderately increased actin-cytoskeleton regulatory genes and decreased expression levels of surfactant-associate genes were observed in IPF patient lungs. By qPCR, we compared the expression levels of actin-cytoskeleton regulatory genes and AT2 biomarker genes in AT2 cells of non-prosthesis and prosthesis implanted *Cdc42* AT2 null mice (mean±S.E.M., n=3). In non-prosthesis implanted *Cdc42* null AT2 cells, the expression levels of actin-cytoskeleton regulatory genes are increased and the expression levels of AT2 biomarker genes are decreased (Figures 24D-24E). The prosthetic implantation not only suppressed the increased expression of actin-cytoskeleton regulatory genes (Figures 24D), but also greatly rescued the decreased expression of AT2 biomarker genes (Figures 24E). Our results support the conclusion that the increased mechanical tension in the overstretched alveoli of *Cdc42* AT2 null lungs positively regulates actin-cytoskeleton regulatory genes and negatively regulates the expression of AT2 biomarker genes. The percentage of survivors among the no-prosthesis-implanted and prosthesis-implanted *Cdc42* AT2 null mice were calculated at post-PNX day 180. Strikingly, by post-PNX 180 days, none of the *Cdc42* AT2 null mice (n=10) that were implanted with a prosthesis died while fully 70% of the no-prosthesis-implanted *Cdc42* AT2 null mice (n=19) were dead by this time point (Figure 24F). The levels of hydroxyproline in the lungs of no-prosthesis-implanted and prosthesis-implanted *Cdc42* AT2 null mice when no-prosthesis-implanted *Cdc42* AT2 null mice reached their end points (mean±S.E.M., n=5) (Figure 24G). H&E staining revealed that little to no lung fibrosis was evident in prosthesis-implanted *Cdc42* AT2 null mice by post-PNX day 180, whereas modest to severe lung fibrosis was observed in all of the surviving *Cdc42* AT2 null mice that were not given a prosthesis following PNX (Figure 24H). Together, these results demonstrate an essential regulatory role for the mechanical tension in driving the development of lung fibrosis in *Cdc42* AT2 null mice. *P<0.05, **P<0.01, ***P<0.001; ****P<0.0001, NS, not significant, Student's t test. Scale bar: 20 µm (B); 2mm (H).

As shown in Figure 20-Figure 22, the loss of *Cdc42* in AT2 cells leads to progressive lung fibrosis following lung injury. The progressive development of lung fibrosis that we observed here is apparently similar to the pathological process that occurs in IPF patients, in which fibrosis initially starts at peripheral regions of the lung before slowly proceeding inwards, eventually affecting entire lung lobes. We show that the loss of alveoli resulting from PNX substantially increases mechanical tension exerted upon the alveolar epithelium. The subsequent efficient regeneration of alveoli that occurs in normal mice eventually reduces the intensity of the mechanical tension to pre-PNX levels; however, as *Cdc42* null AT2 cells are unable to differentiate into AT1 cells and thus cannot regenerate new alveoli, the alveolar epithelium of *Cdc42* null mice continue to experience elevated mechanical tension.

And further, the present invention provides a whole new and inventive method for treating IPF, the progression of which could not be reversed or even slowed down up to now. The present method uses pulmonary prosthesis implanted in the pleural cavity of human beings and is an efficient method for treating IPF without using any drugs.

### References:

1 Wynn, T. Cellular and molecular mechanisms of fibrosis. The Journal of Pathology: A Journal of the Pathological Society of Great Britain and Ireland 214, 199-210 (2008).
2 Wynn, T. A. & Ramalingam, T. R. Mechanisms of fibrosis: therapeutic translation for fibrotic disease. Nature medicine 18, 1028 (2012).
3 Mehal, W. Z., Iredale, J. & Friedman, S. L. Scraping fibrosis: expressway to the core of fibrosis. Nature medicine 17, 552 (2011).
4 Barkauskas, C. E. & Noble, P. W. Cellular mechanisms of tissue fibrosis. 7. New insights into the cellular mechanisms of pulmonary fibrosis. American Journal of Physiology-Cell Physiology 306, C987-C996 (2014).
5 Rock, J. R. et al. Multiple stromal populations contribute to pulmonary fibrosis without evidence for epithelial to mesenchymal transition. Proceedings of the National Academy of Sciences 108, E1475-E1483 (2011).
6 Gross, T. J. & Hunninghake, G. W. Idiopathic pulmonary fibrosis. New England Journal of Medicine 345, 517-525 (2001).
7 Vyalov, S. L., Gabbiani, G. & Kapanci, Y. Rat alveolar myofibroblasts acquire alpha-smooth muscle actin expression during bleomycin-induced pulmonary fibrosis. The American journal of pathology 143, 1754 (1993).
8 King Jr, T. E., Pardo, A. & Selman, M. Idiopathic pulmonary fibrosis. The Lancet 378, 1949-1961 (2011).
9 Plantier, L. et al. Ectopic respiratory epithelial cell differentiation in bronchiolised distal airspaces in idiopathic pulmonary fibrosis. Thorax 66, 651-657 (2011).
10 Steele, M. P. & Schwartz, D. A. Molecular mechanisms in progressive idiopathic pulmonary fibrosis. Annual review of medicine 64, 265-276 (2013).
11 Nogee, L. M. et al. A mutation in the surfactant protein C gene associated with familial interstitial lung disease. New England Journal of Medicine 344, 573-579 (2001).
12 Seibold, M. A. et al. A common MUC5B promoter polymorphism and pulmonary fibrosis. New England Journal of Medicine 364, 1503-1512 (2011).
13 Wang, Y. et al. Genetic defects in surfactant protein A2 are associated with pulmonary fibrosis and lung cancer. The American Journal of Human Genetics 84, 52-59 (2009).
14 Barkauskas, C. E. et al. Type 2 alveolar cells are stem cells in adult lung. The Journal of clinical investigation 123, 3025-3036 (2013).
15 Desai, T. J., Brownfield, D. G. & Krasnow, M. A. Alveolar progenitor and stem cells in lung development, renewal and cancer. Nature 507, 190 (2014).
16 Haies, D. M., Gil, J. & Weibel, E. R. Morphometric study of rat lung cells: I. Numerical and dimensional characteristics of parenchymal cell population. American Review of Respiratory Disease 123, 533-541 (1981).
17 Selman, M. & Pardo, A. Idiopathic pulmonary fibrosis: an epithelial/fibroblastic crosstalk disorder. Respiratory research 3, 3 (2001).
18 Kropski, J. A., Blackwell, T. S. & Loyd, J. E. The genetic basis of idiopathic pulmonary fibrosis. European Respiratory Journal 45, 1717-1727 (2015).
19 Goodwin, A. T. & Jenkins, G. Molecular endotyping of pulmonary fibrosis. Chest 149, 228-237 (2016).
20 Meltzer, E. B. & Noble, P. W. Idiopathic pulmonary fibrosis. Orphanet journal of rare diseases 3, 8, doi:10.1186/1750-1172-3-8 (2008).
21 Richeldi, L., Collard, H. R. & Jones, M. G. Idiopathic pulmonary fibrosis. The Lancet 389, 1941-1952, doi:10.1016/s0140-6736(17)30866-8 (2017).
22 TE, J. K. et al. Idiopathic pulmonary fibrosis. American journal of respiratory and critical care medicine 161, 646-664 (2000).
23 Lynch, D. A. et al. High-resolution computed tomography in idiopathic pulmonary fibrosis: diagnosis and prognosis. American journal of respiratory and critical care medicine 172, 488-493 (2005).
24 Noble, P. W. et al. Pirfenidone in patients with idiopathic pulmonary fibrosis (CAPACITY): two randomised trials. The Lancet 377, 1760-1769 (2011).
25 Raghu, G. et al. An official ATS/ERS/JRS/ALAT statement: idiopathic pulmonary fibrosis: evidence-based guidelines for diagnosis and management. American journal of respiratory and critical care medicine 183, 788-824 (2011).

## Claims

1. Prosthesis for treating pulmonary fibrosis, which is designed to be anchored under the pulmonary lobe and above a diaphragm, and is designed to match with a bottom of the pulmonary lobe, so that the level of mechanical tension on alveolar epithelium is reduced at an end of normal inspiratory, and
wherein the prosthesis comprises a body having an upper side, a lower side and a lateral side extending between the upper side and the lower side, and is in a shape of plate, ellipse, irregular U, arc, conical, scapula, or irregular, so as to occupy a space under the pulmonary lobe.

2. The prosthesis of claim 1, wherein the prosthesis is suitable for occupying at least 1/8, at least 1/4, at least half, at least 3/4, or the whole of a space surrounded by a bottom of the pulmonary lobe, a diaphragm and a wall of pleural cavity.

3. The prosthesis of claim 2, wherein the prosthesis is suitable for being surrounded by pleural fluid.

4. The prosthesis of claim 1, wherein the prosthesis suitable for covering at least 1/8, at least 1/4, at least half, at least 3/4, or the whole of a basal area of the lower pulmonary lobe.

5. The prosthesis of claim 1, wherein the prosthesis upper side is suitable for matching with the bottom of the pulmonary lobe,
wherein the prosthesis lower side is suitable for matching with a diaphragm,
wherein the prosthesis outer lateral side is suitable for matching with a wall of pleural cavity between the bottom of the pulmonary lobe and the diaphragm far from the heart, and/or
wherein the prosthesis inner lateral side is suitable for matching with an outer wall of the lower pulmonary lobe.

6. The prosthesis of claim 1, wherein the prosthesis has a smooth curved profile without edges and corners so as to reduce discomfort and avoid injury to a pleura.

7. The prosthesis of claim 1, wherein the prosthesis comprises a body having an upper side, a lower side, a lateral side extending between the upper side and the lower side, and a medial border approaching the heart, wherein the lateral side is opposite to the medial border, preferably, the prosthesis upper side tapers toward the medial border, and the prosthesis lower side tapers toward the medial border.

8. The prosthesis of claim 1, wherein the lateral side is at a height of 0.5cm-8cm, or,
the lateral side is at a height of 0.5cm, 0.6cm, 0.7cm, 0.8cm, 0.8cm, 0.9cm, 1cm, 2cm, 3cm, 4cm, 5cm, 6cm, 7cm, or 8cm.

9. The prosthesis of claim 1, wherein a thickness of a wall of the prosthesis is between 0.1-4cm, or,
the thickness is 0.1cm, 0.2cm, 0.3cm, 0.4cm, 0.5cm, 0.6cm, 0.7cm, 0.8cm, 0.9cm, 1cm, 1.1cm, 1.2cm, 1.3cm, 1.4cm, 1.5cm, 1.6cm, 1.7cm, 1.8cm, 1.9cm, 2cm, 3cm, or 4cm.

10. The prosthesis of claim 1, wherein the prosthesis is in a shape of arc, and the arc is suitable for matching with a lower edge of a lower pulmonary lobe,
preferably, the arc is suitable for matching with at least 1/8, at least 1/4, or at least 1/2 of the lower edge of the lower pulmonary lobe far from the heart, or suitable for matching with an overall length of the lower edge of the lower pulmonary lobe.

11. The prosthesis of claim 10, wherein the prosthesis is at a height of 1cm-4cm.

12. The prosthesis of claim 10, wherein the lateral side of the prosthesis is at a height of 1cm, 2cm, 3cm, or 4cm.

13. The prosthesis of any one of claims 1-12, wherein the prosthesis is suitable for being placed at a lower side of the pleural cavity, so as to reduce the mechanical tension on the alveolar epithelium at the end of normal inspiratory.

14. The prosthesis of any one of claims 1-12, wherein the prosthesis is suitable for being fixed in a lower side of the pleural cavity,
optionally, the prosthesis is suitable for being sutured to a wall of the pleural cavity, particularly, to a lower portion of the pleural cavity wall, using surgical sutures,
optionally, the prosthesis is suitable for being sutured to the wall of pleural cavity through pinholes on the lateral side.

15. The prosthesis of any one of claims 1-14, wherein the prosthesis is made of soft spongy latex, foam latex, 380 micron hollow fiber, gelatin foam, material, plastic sponge (Ivalon), polythene bag filled with fiberglass, rubber, silicone rubber, silicone gel, carbon materials involving carbon nanotube, grapheme, ultra-light porous carbon, hollow porous carbon, carbon fiber, or carbon titanium alloy.

16. The prosthesis of any one of claims 1-15, wherein the prosthesis is solid, or the prosthesis is a pocket or a sack, filled with fluid or jelly.

17. The prosthesis of any one of claims 1-16, wherein the pulmonary fibrosis is idiopathic pulmonary fibrosis (IPF).

## Patentansprüche

1. Prothese zur Behandlung von Lungenfibrose, dazu ausgelegt, unter dem Lungenlappen und über einem Diaphragma verankert zu werden, und dazu ausgelegt, mit einer Unterseite des Lungenlappens zusammenzupassen, so dass das Niveau an mechanischer Spannung an Alveolarepithel an einem Ende von normaler Inspiration verringert ist, und
wobei die Prothese einen Körper mit einer Oberseite, einer Unterseite und einer lateralen Seite, die zwischen der Oberseite und der Unterseite verläuft, umfasst und in einer Form von Platte, Ellipse, unregelmäßigem U, Bogen, konisch, Scapula oder unregelmäßig vorliegt, um einen Raum unter dem Lungenlappen zu besetzen.

2. Prothese nach Anspruch 1, wobei die Prothese geeignet ist, wenigstens 1/8, wenigstens 1/4, wenigstens die Hälfte, wenigstens 3/4 oder den gesamten Raum, der von einer Unterseite des Lungenlappens, einem Diaphragma und einer Wand der Pleurahöhle umgeben ist, einzunehmen.

3. Prothese nach Anspruch 2, wobei die Prothese geeignet ist, von Pleurafluid umgeben zu sein.

4. Prothese nach Anspruch 1, wobei die Prothese geeignet ist, wenigstens 1/8, wenigstens 1/4, wenigstens die Hälfte, wenigstens 3/4 oder die gesamte Basalfläche des unteren Lungenlappens zu bedecken.

5. Prothese nach Anspruch 1, wobei die Prothesenoberseite geeignet ist, mit der Unterseite des Lungenlappens zusammenzupassen,
wobei die Prothesenunterseite geeignet ist, mit einem Diaphragma zusammenzupassen,
wobei die äußere seitliche Prothesenseite geeignet ist, mit einer Wand einer Pleurahöhle zwischen der Unterseite des Lungenlappens und dem herzfernen Diaphragma zusammenzupassen, und/oder
wobei die innere seitliche Prothesenseite geeignet ist, mit einer Außenwand des unteren Lungenlappens zusammenzupassen.

6. Prothese nach Anspruch 1, wobei die Prothese ein glattes gekrümmtes Profil ohne Kanten und Ecken aufweist, um Beschwerden zu verringern und Verletzungen einer Pleura zu vermeiden.

7. Prothese nach Anspruch 1, wobei die Prothese einen Körper mit einer Oberseite, einer Unterseite, einer lateralen Seite, die sich zwischen der Oberseite und der Unterseite erstreckt, und einer medialen Grenze, die sich dem Herzen nähert, umfasst, wobei die laterale Seite der medialen Grenze gegenüberliegt, wobei sich die Prothesenoberseite vorzugsweise in Richtung zu der medialen Grenze verjüngt und sich die Prothesenunterseite in Richtung zu der medialen Grenze verjüngt.

8. Prothese nach Anspruch 1, wobei die laterale Seite in einer Höhe von 0,5 cm bis 8 cm liegt, oder
die laterale Seite in einer Höhe von 0,5 cm, 0,6 cm, 0,7 cm, 0,8 cm, 0,8 cm, 0,9 cm, 1 cm, 2 cm, 3 cm, 4 cm, 5 cm, 6 cm, 7 cm oder 8 cm liegt.

9. Prothese nach Anspruch 1, wobei eine Dicke einer Wand der Prothese zwischen 0,1 und 4 cm beträgt, oder
die Dicke 0,1cm, 0,2 cm, 0,3 cm, 0,4 cm, 0,5 cm, 0,6 cm, 0,7 cm, 0,8 cm, 0,9 cm, 1cm, 1,1cm, 1,2 cm, 1,3 cm, 1,4 cm, 1,5 cm, 1,6 cm, 1,7 cm, 1,8 cm, 1,9 cm, 2 cm, 3 cm oder 4 cm beträgt.

10. Prothese nach Anspruch 1, wobei die Prothese in einer Bogenform vorliegt und der Bogen geeignet ist, mit einer unteren Kante eines unteren Lungenlappens zusammenzupassen,
vorzugsweise der Bogen geeignet ist, mit wenigstens 1/8, wenigstens 1/4 oder wenigstens 1/2 unteren Kante des unteren Lungenlappens herzfern zusammenzupassen, oder geeignet ist, mit einer gesamten Länge der unteren Kante des unteren Lungenlappens zusammenzupassen.

11. Prothese nach Anspruch 10, wobei die Prothese in einer Höhe von l cm bis 4 cm liegt.

12. Prothese nach Anspruch 10, wobei die laterale Seite der Prothese in einer Höhe von 1 cm, 2 cm, 3 cm oder 4 cm liegt.

13. Prothese nach einem der Ansprüche 1-12, wobei die Prothese geeignet ist, an einer unteren Seite der Pleurahöhle platziert zu werden, um die mechanische Spannung an dem Alveolarepithel am Ende von normaler Inspiration zu verringern.

14. Prothese nach einem der Ansprüche 1-12, wobei die Prothese geeignet ist, in einer unteren Seite der Pleurahöhle fixiert zu werden,
gegebenenfalls die Prothese geeignet ist, mit einer Wand der Pleurahöhle, insbesondere mit einem unteren Abschnitt der Wand der Pleurahöhle, durch chirurgische Nähte vernäht zu werden,
gegebenenfalls die Prothese geeignet ist, durch Nahtlöcher an der lateralen Seite an die Wand der Pleurahöhle genäht zu werden.

15. Prothese nach einem der Ansprüche 1-14, wobei die Prothese aus weichem schwammigem Latex, Schaumlatex, 380-Mikrometer-Hohlfaser, Gelatineschaum, Material, Kunststoffschwamm (Ivalon), Glasfaser-gefülltem Polythenbeutel, Gummi, Silikonkautschuk, Silikongel, Kohlenstoffmaterialien unter Beteiligung von Kohlenstoffnanoröhrchen, Graphen, ultraleichtem porösem Kohlenstoff, hohlem porösem Kohlenstoff, Kohlenstofffaser oder Kohlenstofftitanlegierung besteht.

16. Prothese nach einem der Ansprüche 1-15, wobei die Prothese fest ist oder die Prothese eine Tasche oder ein Beutel ist, die mit Fluid oder Gallerte gefüllt ist.

17. Prothese nach einem der Ansprüche 1-16, wobei die Lungenfibrose idiopathische Lungenfibrose (IPF) ist.

## Revendications

1. Prothèse pour le traitement de la fibrose pulmonaire, qui est conçue pour être ancrée sous le lobe pulmonaire et au-dessus d'un diaphragme, et est conçue pour correspondre à une partie inférieure du lobe pulmonaire, de sorte que le niveau de tension mécanique sur l'épithélium alvéolaire soit réduit à la fin d'une inspiration normale, et
dans lequel la prothèse comprend un corps ayant un côté supérieur, un côté inférieur et un côté latéral s'étendant entre le côté supérieur et le côté inférieur, et est de forme plate, elliptique, en U irrégulier, arquée, conique, scapulaire ou irrégulière, de manière à occuper un espace sous le lobe pulmonaire.

2. Prothèse selon la revendication 1, la prothèse étant appropriée pour occuper au moins 1/8, au moins 1/4, au moins la moitié, au moins les 3/4, ou la totalité d'un espace entouré par une partie inférieure du lobe pulmonaire, un diaphragme et une paroi de cavité pleurale.

3. Prothèse selon la revendication 2, la prothèse étant appropriée pour être entourée de liquide pleural.

4. Prothèse selon la revendication 1, la prothèse étant appropriée pour recouvrir au moins 1/8, au moins 1/4, au moins la moitié, au moins les 3/4, ou la totalité d'une aire basale du lobe pulmonaire inférieur.

5. Prothèse selon la revendication 1, dans laquelle le côté supérieur de la prothèse est approprié pour correspondre avec la partie inférieure du lobe pulmonaire,
dans laquelle le côté inférieur de prothèse est approprié pour correspondre à un diaphragme,
dans laquelle le côté latéral externe de la prothèse est approprié pour correspondre à une paroi de cavité pleurale entre la partie inférieure du lobe pulmonaire et le diaphragme à distance du cœur, et/ou
dans laquelle le côté latéral interne de la prothèse est approprié pour correspondre à une paroi externe du lobe pulmonaire inférieur.

6. Prothèse selon la revendication 1, dans laquelle la prothèse a un profil incurvé lisse sans bords ni coins de manière à réduire l'inconfort et à éviter de blesser la plèvre.

7. Prothèse selon la revendication 1, la prothèse comprenant un corps ayant un côté supérieur, un côté inférieur, un côté latéral s'étendant entre le côté supérieur et le côté inférieur, et une bordure médiale s'approchant du cœur, dans laquelle le côté latéral est opposé à la bordure médiale, de préférence, le côté supérieur de la prothèse s'effile vers la bordure médiale, et le côté inférieur de la prothèse s'effile vers la bordure médiale.

8. Prothèse selon la revendication 1, dans laquelle le côté latéral est à une hauteur de 0,5 cm à 8 cm, ou,
le côté latéral est à une hauteur de 0,5 cm, 0,6 cm, 0,7 cm, 0,8 cm, 0,8 cm, 0,9 cm, 1 cm, 2 cm, 3 cm, 4 cm, 5 cm, 6 cm, 7 cm ou 8 cm.

9. Prothèse selon la revendication 1, dans laquelle une épaisseur d'une paroi de la prothèse est entre 0,1 et 4 cm, ou,
l'épaisseur est 0,1 cm, 0,2 cm, 0,3 cm, 0,4 cm, 0,5 cm, 0,6 cm, 0,7 cm, 0,8 cm, 0,9 cm, 1 cm, 1,1 cm, 1,2 cm, 1,3 cm, 1,4 cm, 1,5 cm, 1,6 cm, 1,7 cm, 1,8 cm, 1,9 cm, 2 cm, 3 cm ou 4 cm.

10. Prothèse selon la revendication 1, la prothèse ayant une forme d'arc, et l'arc étant approprié pour correspondre à un bord inférieur d'un lobe pulmonaire inférieur,
de préférence, l'arc étant approprié pour correspondre à au moins 1/8, au moins 1/4, ou au moins 1/2 du bord inférieur du lobe pulmonaire inférieur à distance du cœur, ou approprié pour correspondre à une longueur totale du bord inférieur du lobe pulmonaire inférieur.

11. Prothèse selon la revendication 10, la prothèse étant à une hauteur de l cm à 4 cm.

12. Prothèse selon la revendication 10, dans laquelle le côté latéral de la prothèse est à une hauteur de 1 cm, 2 cm, 3 cm ou 4 cm.

13. Prothèse selon l'une quelconque des revendications 1 à 12, la prothèse étant appropriée pour être placée au niveau d'un côté inférieur de la cavité pleurale, de manière à réduire la tension mécanique sur l'épithélium alvéolaire à la fin d'une inspiration normale.

14. Prothèse selon l'une quelconque des revendications 1 à 12, la prothèse étant appropriée pour être fixée dans un côté inférieur de la cavité pleurale, éventuellement, la prothèse étant appropriée pour être suturée à une paroi de la cavité pleurale, en particulier, à une partie inférieure de la paroi de la cavité pleurale, à l'aide de sutures chirurgicales, éventuellement, la prothèse étant appropriée pour être suturée à la paroi de la cavité pleurale à travers des petits trous sur le côté latéral.

15. Prothèse selon l'une quelconque des revendications 1 à 14, la prothèse étant réalisée en latex spongieux mou, latex expansé, fibre creuse de 380 micromètres, mousse de gélatine, matériau, éponge plastique (Ivalon), sac en polythène rempli de fibre de verre, caoutchouc, caoutchouc silicone, gel silicone, matériaux carbonés comprenant nanotube de carbone, graphène, carbone poreux ultraléger, carbone poreux creux, fibre de carbone, ou alliage carbone-titane.

16. Prothèse selon l'une quelconque des revendications 1 à 15, la prothèse étant solide, ou la prothèse étant une poche ou un sac, rempli de fluide ou de gelée.

17. Prothèse selon l'une quelconque des revendications 1 à 16, dans laquelle la fibrose pulmonaire est une fibrose pulmonaire idiopathique (FPI).
